(19) Europäisches Patentamt
European Patent Office
Office européen des brevets

(11) EP 4 810 036 A1

(12) EUROPEAN PATENT APPLICATION
published in accordance with Art. 153(4) EPC

(43) Date of publication:
**23.09.2026 Bulletin 2026/39**

(21) Application number: **24890785.9**

(22) Date of filing: **14.11.2024**

(51) International Patent Classification (IPC):
***A61K 31/407*** *(2006.01)* ***A61K 9/48*** *(2006.01)*
***A61K 9/20*** *(2006.01)* ***A61P 37/02*** *(2006.01)*

(52) Cooperative Patent Classification (CPC):
**A61K 9/20; A61K 9/48; A61K 31/407; A61P 11/00; A61P 31/06; A61P 37/02**

(86) International application number:
**PCT/CN2024/132140**

(87) International publication number:
**WO 2025/103430 (22.05.2025 Gazette 2025/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.11.2023 CN 202311516190**

(71) Applicant: **Tianjin Hemay Pharmaceutical Co., Ltd.**
**Tianjin 300308 (CN)**

(72) Inventors:
- **ZHANG, Hesheng**
  **Tianjin 300308 (CN)**
- **ZENG, Guanghuai**
  **Tianjin 300308 (CN)**
- **FU, Wei**
  **Tianjin 300308 (CN)**
- **LV, Jingjing**
  **Tianjin 300308 (CN)**
- **LIU, Xiuping**
  **Tianjin 300308 (CN)**

(74) Representative: **ABG Intellectual Property Law, S.L.**
**Avenida de Burgos, 16D**
**Edificio Euromor**
**28036 Madrid (ES)**

(54) **PREVENTION OR TREATMENT OF SJÖGREN'S SYNDROME**

(57) A method for preventing or treating Sjögren's syndrome. The method comprises administering to a subject in need an effective amount of N-[5-[1-(3-ethoxy-4-methoxyphenyl)-2-(methylsulfonyl) ethyl]-4,6-dioxo-5,6-dihydro-4H-thieno[3,4-c]pyrrole-1-yl]acetamide, a stereoisomer thereof or a pharmaceutically acceptable salt thereof.

(I).



Processed by Luminess, 75001 PARIS (FR)

## Description

### CROSS-REFERENCE TO RELATED APPLICATION

The present disclosure claims the full benefits of Chinese Patent Application No. 202311516190.8, filed on November 14, 2023, with the State Intellectual Property Office of the People's Republic of China, entitled "Treatment of Sjögren's Syndrome", which is incorporated herein by reference in its entirety.

### TECHNICAL FIELD

The present disclosure generally relates to the field of medicine, and more specifically, to the prevention or treatment of Sjögren's syndrome.

### BACKGROUND

Sjögren's syndrome (SS) is a systemic autoimmune disease characterized by lymphocyte proliferation and progressive damage to exocrine glands, with features including lymphocytic infiltration of exocrine glands and epithelial cells at multiple sites, as well as positivity for serum autoantibodies such as anti-Sjögren's syndrome antibodies. It primarily attacks the lacrimal glands and salivary glands, resulting in xerophthalmia and xerostomia. In addition, Sjögren's syndrome can also affect numerous extraglandular organs and systems.

The exact etiology and pathogenesis of Sjögren's syndrome remain unclear. At present, it is generally believed to result from immune dysfunction induced by many factors, including genetics, viral infection, and sex hormone abnormalities. It exhibits a wide spectrum of clinical manifestations, ranging from impaired secretory function of the salivary and lacrimal glands to multi-organ and multi-system involvement with systemic symptoms, and even complicated lymphoma. There are no satisfactory treatments for Sjögren's syndrome. No evidence-based effective medications are available for dryness, fatigue, pain, or damage to visceral organs. Most of the drugs used in clinical practice are empirical therapies.

Patients suffering from Sjögren's syndrome often have impaired or disordered immune function, thereby increasing a risk of infection with pathogens. Long-term administration of currently available therapeutic drugs for Sjögren's syndrome may further reduce immune competence in patients suffering from Sjögren's syndrome, thereby further increasing the risk of infection with pathogens. Among such pathogens, *Mycobacterium tuberculosis* is a common infectious bacteria encountered during treatment. Approximately one-third of the global population is infected with *Mycobacterium tuberculosis*, and 90% of infected individuals can carry the pathogen persistently for a long time and develop latent tuberculosis infection. Latent tuberculosis infection serves as a breeding ground for the development of active tuberculosis, and patients with latent tuberculosis infection exhibit highly uncertain disease progression outcome, representing a concealed critical risk in tuberculosis control. Impaired immune function in patients with latent tuberculosis infection tends to facilitate the progression from latent tuberculosis to active tuberculosis. It is of profound significance and impact to focus on the special patient population with latent tuberculosis infection during treatment, provide appropriate therapeutic regimens for patients with Sjögren's syndrome as well as patients with Sjögren's syndrome accompanied by latent tuberculosis infection or a history of tuberculosis, improve efficacy, reduce adverse reactions and the potential risk of progression to active tuberculosis, and strive for greater benefits for such patients.

### SUMMARY OF THE INVENTION

In one aspect, the present disclosure relates to a method for preventing or treating Sjögren's syndrome, comprising administering to a subject in need thereof a prophylactically or therapeutically effective amount of a compound of formula (I), a stereoisomer thereof, or a pharmaceutically acceptable salt thereof,

Formula (I).

In another aspect, the present disclosure relates to a compound of formula (I), a stereoisomer thereof, or a pharmaceutically acceptable salt thereof for use in the prevention or treatment of Sjögren's syndrome in a subject,

Formula (I).

In still another aspect, the present disclosure relates to a use of a compound of formula (I), a stereoisomer thereof, or a pharmaceutically acceptable salt thereof in the preparation of a medicament for the prevention or treatment of Sjögren's syndrome in a subject,

Formula (I).

In yet another aspect, the present disclosure relates to a pharmaceutical composition for use in the prevention or treatment of Sjögren's syndrome in a subject, comprising a compound of formula (I), a stereoisomer thereof or a pharmaceutically acceptable salt thereof, and a pharmaceutically acceptable carrier, diluent or excipient,

Formula (I).

**DETAILED** DESCRIPTION OF THE INVENTION

In the following description, certain specific details are set forth to provide a thorough understanding of various embodiments of the disclosure. However, a person skilled in the related art would recognize that the embodiments can still be practiced by using other methods, components, materials, etc., without employing one or more of these specific details.

Unless required otherwise in the present application, throughout the specification and the appended claims, the words "comprising," "including," "containing," and "having" are to be construed in an open, inclusive sense, that is, as "including but not limited to".

When used in the present disclosure and the appended claims, a singular designation without a quantity indication includes a plural designation unless stated otherwise in the context.

Reference throughout this specification to "one embodiment", "an embodiment", "in another embodiment", and "in some embodiments" means that a specific reference element, structure, or characteristic described in connection with the embodiment is included in at least one embodiment of the present disclosure. Thus, the appearances of the phrases "in one embodiment", "in an embodiment", "in another embodiment", and "in some embodiments" in various places throughout this specification are not necessarily all referring to the same embodiment. Furthermore, specific elements, structures, or features may be combined in any suitable manner in one or more embodiments.

It should be understood that the singular articles "a", "an" and "the" used in the specification and the appended claims of the present disclosure include plural references unless the context clearly dictates otherwise. Therefore, for example, reference to a pharmaceutical composition comprising "an excipient" includes a pharmaceutical composition comprising one excipient, or a pharmaceutical composition comprising two or more excipients.

Definition

Therefore, unless stated otherwise, the following terms used in the present specification and appended claims shall have the following meanings.

In the present disclosure, the term "Sjögren's syndrome (SS)" refers to an autoimmune disease that causes glands to produce less water than normal. Sjögren's syndrome results in chronic (long-term) dryness throughout the body, especially in the eyes and mouth.

In the present disclosure, there are two commonly used classification methods for adverse events occurring in clinical trials. One classification method categorizes adverse reactions as mild, moderate, and severe. "Mild" means that said adverse reaction is usually transient, generally does not affect the subjects' activities of daily living, and may require only minimal treatment. "Moderate" means that said adverse reaction causes discomfort to the subjects, affects activities of daily living, and usually requires treatment for relief, but does not pose a risk of substantial or permanent harm to the subject. "Severe" means that said adverse reaction significantly affects the subjects' activities of daily living or seriously deteriorates their clinical condition, requiring intensive treatment and intervention. The other classification method categorizes adverse events as Grade 1 to Grade 5 with reference to the Common Terminology Criteria for Adverse Events (CTCAE). Grade 1: mild, no or mild clinical symptoms; only clinical or diagnostic observations; no treatment required. Grade 2: moderate, requires minor, local or non-invasive treatment, limitation in age-appropriate instrumental Activities of Daily Living (ADL). Instrumental Activities of Daily Living refer to cooking, clothing purchasing, telephone use, financial management, etc. Grade 3: severe or medically significant, but not immediately life-threatening; results in hospitalization or prolonged hospitalization; disability; limitations in basic activities of daily living. Basic Activities of Daily Living refer to bathing, dressing, eating, personal hygiene, medication administration, etc., without bedridden status. Grade 4: life-threatening, requires urgent intervention. Grade 5: death related to the adverse event.

In the present disclosure, the term "HADS" refers to the Hospital Anxiety and Depression Scale, which is used for screening anxiety and depressive symptoms in patients in general hospitals. Featuring simplicity, conciseness and

practicality, the questionnaire has been extensively studied in routine medical settings. The questionnaire contains 7 anxiety-related items (A) and 7 depression-related items (D). Responses to all items are rated on a 4-point scale (0-3 points). The total score for anxiety or depression is the sum of the scores for individual responses to the anxiety or depression-related items, ranging from 0 to 21 points. Scores of 0 to 7 indicate the absence of anxiety or depression; scores of 8 to 10 indicate mild anxiety or depression; scores of 11 to 14 indicate moderate anxiety or depression; and scores of 15 to 21 indicate severe anxiety or depression.

In the present disclosure, the term "latent tuberculosis infection (LTBI)" means that an organism is infected with *Mycobacterium tuberculosis,* without development of clinical tuberculosis, absence of clinical symptoms, and no bacteriological or imaging evidence of active tuberculosis. Diagnostic criteria for latent tuberculosis include a positive T-Cell Spot Assay (T-SPOT) for *Mycobacterium tuberculosis* infection and no clinical manifestations of active tuberculosis.

In the present disclosure, the term "activation" refers to a patient's *Mycobacterium tuberculosis* infection status transition from latent tuberculosis infection to active tuberculosis infection; that is, the patient's *Mycobacterium tuberculosis* infection status progresses to an active stage, and the patient is in an active tuberculosis state, and requires standardized and intensive treatment.

In the present disclosure, the term "FCA" refers to Freund's complete adjuvant.

In the present disclosure, the term "compound of formula (I)" refers to N-[5-[1-(3-ethoxy-4-methoxyphenyl)-2-(methylsulfonyl)ethyl]-4,6-dioxo-5,6-dihydro-4H-thieno[3,4-c]pyrrol-1-yl]acetamide, the structural formula of which is shown below:

.

In the present disclosure, the term "a stereoisomer of formula (I)" refers to (S)-N-[5-[1-(3-ethoxy-4-methoxyphenyl)-2-(methylsulfonyl)ethyl]-4,6-dioxo-5,6-dihydro-4H-thieno[3,4-c]pyrrol-1-yl]acetamide, the structural formula of which is shown below:

.

In the present disclosure, the term "pharmaceutically acceptable" means that a carrier, vehicle, diluent, excipient, and/or salt is compatible with other components of the formulation and not deleterious to the recipient thereof.

In the present disclosure, the term "pharmaceutically acceptable carrier, diluent, or excipient" includes, but is not limited to, any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, or emulsifier in various forms approved by the United States Food and Drug Administration for use in humans or animals, which has no adverse effects on the pharmaceutical composition.

In the present disclosure, the term "carrier" is defined as a compound that facilitates the introduction of a compound into a cell or tissue. For example, dimethyl sulfoxide (DMSO) is commonly used as a carrier due to its ability to readily introduce certain organic compounds into a cell or tissue of an organism.

In the present disclosure, the term "pharmaceutically acceptable salt" includes "an acceptable acid addition salt" and "an acceptable base addition salt".

In the present disclosure, the term "acceptable acid addition salt" refers to those salts which retain the biological

effectiveness and properties of the free bases, which are biologically or otherwise desirable, and are formed with inorganic or organic acids. The inorganic acids include, but are not limited to, hydrochloric acid, hydrobromic acid, sulfuric acid, nitric acid, phosphoric acid, and the like. The organic acid include, but are not limited to, acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor-10-sulfonic acid, capric acid, caproic acid, caprylic acid, carbonic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, undecylenic acid, and the like.

In the present disclosure, the term "acceptable base addition salt" refers to those salts which retain the biological effectiveness and properties of the free acids, which are biologically or otherwise desirable. These salts are prepared from addition of an inorganic base or an organic base to the free acid. Salts derived from inorganic bases include, but are not limited to, the sodium, potassium, lithium, ammonium, calcium, magnesium, iron, zinc, copper, manganese, aluminum salts and the like. In certain embodiments, inorganic salts are the ammonium, sodium, potassium, calcium, and magnesium salts. Salts derived from organic bases include, but are not limited to, salts of primary, secondary, and tertiary amines, substituted amines including naturally occurring substituted amines, cyclic amines and basic ion exchange resins, such as ammonia, isopropylamine, trimethylamine, diethylamine, triethylamine, tripropylamine, diethanolamine, ethanolamine, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucamine, theobromine, triethanolamine, tromethamine, purines, piperazine, piperidine, N-ethylpiperidine, polyamine resins and the like. In certain embodiments, organic bases are isopropylamine, diethylamine, ethanolamine, trimethylamine, dicyclohexylamine, choline and caffeine.

In the present disclosure, the term "mammal" refers to an animal including, for example, a dog, cat, cow, sheep, horse, and human. In some embodiments, the mammal is a human.

In the present disclosure, the term "pharmaceutical composition" refers to a formulation of a compound of formula (I), a stereoisomer thereof, or a pharmaceutically acceptable salt thereof described in the present disclosure and a medium generally accepted in the art for the delivery of the biologically active compound to mammals, e.g., humans. Such a medium includes all pharmaceutically acceptable carriers, diluents or excipients therefor.

In the present disclosure, the term "therapeutically effective amount" refers to an amount of a compound or a combination of compounds that ameliorates, attenuates, or eliminates a specific disease or condition and the symptoms thereof, or prevents or delays such specific disease or condition or the onset of the symptoms thereof. The amount of the compound of formula (I) or a stereoisomer thereof described in the present disclosure which constitutes a "therapeutically effective amount" will vary depending on the compound, the condition and its severity, and the age and weight of the mammal to be treated, but the amount of the compound described in the present disclosure can be determined routinely by one of ordinary skill in the art having regard to his own knowledge and to this disclosure.

As used in the present disclosure, "prophylactic treatment" or "prevention" encompasses preventing the occurrence of a disease or condition in a mammal (e.g., a human), especially where such mammal (e.g., a human) is susceptible to said disease or condition but has not yet been diagnosed as suffering therefrom.

As used in the present disclosure, "treating" or "treatment" covers the treatment of the disease or condition of interest in a mammal, such as a human, having the disease or disorder of interest, and includes:

(i) inhibiting the disease or condition, i.e., arresting its development; or
(ii) relieving the disease or condition, i.e., causing regression or resulting in no progression of the disease or condition.

In the present disclosure, the term "unit dose" refers to a dosage for a single application. For example, for a tablet, a unit dose refers to the dosage of one tablet.

Detailed Description

In one aspect, the present disclosure relates to a method for preventing or treating Sjögren's syndrome, comprising administering to a subject in need thereof a prophylactically or therapeutically effective amount of a compound of formula (I), a stereoisomer thereof, or a pharmaceutically acceptable salt thereof,

Formula (I).

In some embodiments, the compound of formula (I), a stereoisomer thereof, or a pharmaceutically acceptable salt thereof is administered to the subject daily at a dose of 30 mg to 180 mg.
In some embodiments, the compound of formula (I), a stereoisomer thereof, or a pharmaceutically acceptable salt thereof is administered to the subject daily at a dose of 30 mg, 40 mg, 45 mg, 50 mg, 60 mg, 70 mg, 80 mg, 90 mg, or 100 mg, 120 mg, 130 mg, 140 mg, 150 mg, 160 mg, 170 mg or 180 mg.
In some embodiments, the compound of formula (I), a stereoisomer thereof, or a pharmaceutically acceptable salt thereof is administered to the subject daily at a dose of 60 mg to 150 mg.
In some embodiments, the compound of formula (I), a stereoisomer thereof, or a pharmaceutically acceptable salt thereof is administered to the subject daily at a dose of 30 mg, 60 mg, 120 mg or 150 mg.
In some embodiments, the compound of formula (I), a stereoisomer thereof, or a pharmaceutically acceptable salt thereof is administered to the subject daily at a dose of 150 mg.
In some embodiments, the compound of formula (I), a stereoisomer thereof, or a pharmaceutically acceptable salt thereof is administered to the subject daily at a dose of 120 mg.
In some embodiments, the compound of formula (I), a stereoisomer thereof, or a pharmaceutically acceptable salt thereof is administered to the subject at least once daily.
In some embodiments, the compound of formula (I), a stereoisomer thereof, or a pharmaceutically acceptable salt thereof is administered to the subject at least twice daily.
In some embodiments, the compound of formula (I), a stereoisomer thereof, or a pharmaceutically acceptable salt thereof is administered to the subject twice daily.
In some embodiments, the compound of formula (I), a stereoisomer thereof, or a pharmaceutically acceptable salt thereof is administered to the subject three times daily.
In some embodiments, the compound of formula (I), a stereoisomer thereof, or a pharmaceutically acceptable salt thereof is administered to the subject twice daily at a dose of 30 mg to 75 mg each time.
In some embodiments, the compound of formula (I), a stereoisomer thereof, or a pharmaceutically acceptable salt thereof is administered to the subject twice daily at a dose of 30 mg to 60 mg each time.
In some embodiments, the compound of formula (I), a stereoisomer thereof, or a pharmaceutically acceptable salt thereof has a unit dosage of 2.5 mg to 150 mg.
In some embodiments, an exemplary unit dosage of the compound of formula (I), a stereoisomer thereof, or a pharmaceutically acceptable salt thereof of the present disclosure includes, but is not limited to, 2.5 mg, 5 mg, 7.5 mg, 10 mg, 15 mg, 20 mg, 30 mg, 40 mg, 45 mg, 50 mg, 60 mg, 70 mg, 75 mg, 80 mg, 90 mg, 100 mg, 110 mg, 120 mg, 130 mg, 140 mg or 150 mg.
In some embodiments, the compound of formula (I), a stereoisomer thereof, or a pharmaceutically acceptable salt thereof has a unit dosage of 15 mg to 150 mg.
In some embodiments, the compound of formula (I), the stereoisomer thereof or the pharmaceutically acceptable salt thereof has a unit dosage of 15 mg, 30 mg, 45 mg, 60 mg, 75 mg, 120 mg or 150 mg.
In some embodiments, the compound of formula (I), a stereoisomer thereof, or a pharmaceutically acceptable salt thereof is administered to the subject in a tablet or capsule form.
In some embodiments, the compound of formula (I), a stereoisomer thereof, or a pharmaceutically acceptable salt thereof is orally administered to the subject.
In some embodiments, an exemplary subject in the present disclosure includes, but is not limited to, a mammal.
In some embodiments, an exemplary mammal in the present disclosure includes, but is not limited to, a dog, cat, cow, sheep, horse, and human.
In some embodiments, the subject is a human.
In some embodiments, the subject suffers from latent tuberculosis infection.
In some embodiments, the subject has a medical history of pulmonary tuberculosis infection, tuberculosis, and/or

tuberculous pleurisy.

In some embodiments, the Sjögren's syndrome of the present disclosure is primary Sjögren's syndrome.

In some embodiments, the Sjögren's syndrome of the present disclosure is secondary Sjögren's syndrome.

In another aspect, the present disclosure relates to a compound of formula (I), a stereoisomer thereof, or a pharmaceutically acceptable salt thereof for use in the prevention or treatment of Sjögren's syndrome in a subject,

O
O
S
O
N
S
NH
O
O
O
O

Formula (I).

In some embodiments, the compound of formula (I), a stereoisomer thereof, or a pharmaceutically acceptable salt thereof is administered to the subject daily at a dose of 30 mg to 180 mg.

In some embodiments, the compound of formula (I), a stereoisomer thereof, or a pharmaceutically acceptable salt thereof is administered to the subject daily at a dose of 30 mg, 40 mg, 45 mg, 50 mg, 60 mg, 70 mg, 80 mg, 90 mg, 100 mg, 120 mg, 130 mg, 140 mg, 150 mg, 160 mg, 170 mg or 180 mg.

In some embodiments, the compound of formula (I), a stereoisomer thereof, or a pharmaceutically acceptable salt thereof is administered to the subject daily at a dose of 60 mg to 150 mg.

In some embodiments, the compound of formula (I), a stereoisomer thereof or a pharmaceutically acceptable salt thereof is administered to the subject daily at a dose of 30 mg, 60 mg, 120 mg or 150 mg.

In some embodiments, the compound of formula (I), a stereoisomer thereof, or a pharmaceutically acceptable salt thereof is administered to the subject daily at a dose of 150 mg.

In some embodiments, the compound of formula (I), a stereoisomer thereof, or a pharmaceutically acceptable salt thereof is administered to the subject daily at a dose of 120 mg.

In some embodiments, the compound of formula (I), a stereoisomer thereof, or a pharmaceutically acceptable salt thereof is administered to the subject at least once daily.

In some embodiments, the compound of formula (I), a stereoisomer thereof, or a pharmaceutically acceptable salt thereof is administered to the subject at least twice daily.

In some embodiments, the compound of formula (I), a stereoisomer thereof, or a pharmaceutically acceptable salt thereof is administered to the subject twice daily.

In some embodiments, the compound of formula (I), a stereoisomer thereof, or a pharmaceutically acceptable salt thereof is administered to the subject three times daily.

In some embodiments, the compound of formula (I), a stereoisomer thereof, or a pharmaceutically acceptable salt thereof is administered to the subject twice daily at a dose of 30 mg to 75 mg each time.

In some embodiments, the compound of formula (I), a stereoisomer thereof, or a pharmaceutically acceptable salt thereof is administered to the subject twice daily at a dose of 30 mg to 60 mg each time.

In some embodiments, the compound of formula (I), a stereoisomer thereof, or a pharmaceutically acceptable salt thereof has a unit dosage of 2.5 mg to 150 mg.

In some embodiments, an exemplary unit dosage of the compound of formula (I), a stereoisomer thereof, or a pharmaceutically acceptable salt thereof of the present disclosure includes, but is not limited to, 2.5 mg, 5 mg, 7.5 mg, 10 mg, 15 mg, 20 mg, 30 mg, 40 mg, 45 mg, 50 mg, 60 mg, 70 mg, 75 mg, 80 mg, 90 mg, 100 mg, 110 mg, 120 mg, 130 mg, 140 mg or 150 mg.

In some embodiments, the compound of formula (I), a stereoisomer thereof, or a pharmaceutically acceptable salt thereof has a unit dosage of 15 mg to 150 mg.

In some embodiments, an exemplary unit dosage of the compound of formula (I), a stereoisomer thereof, or a pharmaceutically acceptable salt thereof of the present disclosure includes, but is not limited to, 15 mg, 30 mg, 45 mg, 60 mg, 75 mg, 120 mg or 150 mg.

In some embodiments, the compound of formula (I), a stereoisomer thereof, or a pharmaceutically acceptable salt thereof is administered to the subject in a tablet or a capsule form.

In some embodiments, the compound of formula (I), a stereoisomer thereof, or a pharmaceutically acceptable

salt thereof is orally administered to the subject.

In some embodiments, an exemplary subject in the present disclosure includes, but is not limited to, a mammal.

In some embodiments, an exemplary mammal in the present disclosure includes, but is not limited to, a dog, cat, cow, sheep, horse and human.

In some embodiments, the subject is a human.

In some embodiments, the subject suffers from latent tuberculosis infection.

In some embodiments, the subject has a medical history of pulmonary tuberculosis infection, tuberculosis, and/or tuberculous pleurisy.

In some embodiments, the Sjögren's syndrome of the present disclosure is primary Sjögren's syndrome.

In some embodiments, the Sjögren's syndrome of the present disclosure is secondary Sjögren's syndrome.

In some embodiments, the compound of formula (I), a stereoisomer thereof, or a pharmaceutically acceptable salt thereof of the present disclosure can be used in the prevention or treatment of Sjögren's syndrome in a subject suffering from latent tuberculosis infection, and latent tuberculosis infection is not reactivated after treatment.

In some embodiments, the compound of formula (I), a stereoisomer thereof, or a pharmaceutically acceptable salt thereof of the present disclosure can be used in the prevention or treatment of Sjögren's syndrome in a subject having a medical history of pulmonary tuberculosis infection, tuberculosis, and/or tuberculous pleurisy, and pulmonary tuberculosis infection, tuberculosis, and/or tuberculous pleurisy is/are not reactivated after treatment.

In some embodiments, the compound of formula (I), a stereoisomer thereof, or a pharmaceutically acceptable salt thereof of the present disclosure does not require dose titration when used in the prevention or treatment of Sjögren's syndrome.

In some embodiments, the compound of formula (I), a stereoisomer thereof, or a pharmaceutically acceptable salt thereof of the present disclosure alleviates xerostomia in the subject when used in the prevention or treatment of Sjögren's syndrome.

In some embodiments, the compound of formula (I), a stereoisomer thereof, or a pharmaceutically acceptable salt thereof of the present disclosure alleviates xerophthalmia in the subject when used in the prevention or treatment of Sjögren's syndrome.

In some embodiments, the compound of formula (I), a stereoisomer thereof, or a pharmaceutically acceptable salt thereof of the present disclosure improves the histopathology of minor salivary glands in the subject when used in the prevention or treatment of Sjögren's syndrome.

In some embodiments, the compound of formula (I), a stereoisomer thereof, or a pharmaceutically acceptable salt thereof of the present disclosure does not induce or exacerbate anxiety in the subject when used in the prevention or treatment of Sjögren's syndrome.

In some embodiments, the compound of formula (I), a stereoisomer thereof, or a pharmaceutically acceptable salt thereof of the present disclosure does not induce or exacerbate depression in the subject when used in the prevention or treatment of Sjögren's syndrome.

In still another aspect, the present disclosure relates to the use of a compound of formula (I), a stereoisomer thereof, or a pharmaceutically acceptable salt thereof in the preparation of a medicament for the prevention or treatment of Sjögren's syndrome in a subject,

Formula (I).

In some embodiments, the compound of formula (I), a stereoisomer thereof, or a pharmaceutically acceptable salt thereof is administered to the subject daily at a dose of 30 mg to 180 mg.

In some embodiments, the compound of formula (I), a stereoisomer thereof, or a pharmaceutically acceptable salt thereof is administered to the subject daily at a dose of 30 mg, 40 mg, 45 mg, 50 mg, 60 mg, 70 mg, 80 mg, 90 mg, 100 mg, 120 mg, 130 mg, 140 mg, 150 mg, 160 mg, 170 mg or 180 mg.

In some embodiments, the compound of formula (I), a stereoisomer thereof, or a pharmaceutically acceptable

salt thereof is administered to the subject daily at a dose of 60 mg to 150 mg.

In some embodiments, the compound of formula (I), a stereoisomer thereof, or a pharmaceutically acceptable salt thereof is administered to the subject daily at a dose of 30 mg, 60 mg, 120 mg or 150 mg.

In some embodiments, the compound of formula (I), a stereoisomer thereof, or a pharmaceutically acceptable salt thereof is administered to the subject daily at a dose of 150 mg.

In some embodiments, the compound of formula (I), a stereoisomer thereof, or a pharmaceutically acceptable salt thereof is administered to the subject daily at a dose of 120 mg.

In some embodiments, the compound of formula (I), a stereoisomer thereof, or a pharmaceutically acceptable salt thereof is administered to the subject at least once daily.

In some embodiments, the compound of formula (I), a stereoisomer thereof, or a pharmaceutically acceptable salt thereof is administered to the subject at least twice daily.

In some embodiments, the compound of formula (I), a stereoisomer thereof, or a pharmaceutically acceptable salt thereof is administered to the subject twice daily.

In some embodiments, the compound of formula (I), a stereoisomer thereof, or a pharmaceutically acceptable salt thereof is administered to the subject three times daily.

In some embodiments, the compound of formula (I), a stereoisomer thereof, or a pharmaceutically acceptable salt thereof is administered to the subject twice daily at a dose of 30 mg to 75 mg each time.

In some embodiments, the compound of formula (I), a stereoisomer thereof, or a pharmaceutically acceptable salt thereof is administered to the subject twice daily at a dose of 30 mg to 60 mg each time.

In some embodiments, the compound of formula (I), a stereoisomer thereof, or a pharmaceutically acceptable salt thereof has a unit dosage of 2.5 mg to 150 mg.

In some embodiments, an exemplary unit dosage of the compound of formula (I), a stereoisomer thereof, or a pharmaceutically acceptable salt thereof of the present disclosure includes, but is not limited to, 2.5 mg, 5 mg, 7.5 mg, 10 mg, 15 mg, 20 mg, 30 mg, 40 mg, 45 mg, 50 mg, 60 mg, 70 mg, 75 mg, 80 mg, 90 mg, 100 mg, 110 mg, 120 mg, 130 mg, 140 mg or 150 mg.

In some embodiments, the compound of formula (I), a stereoisomer thereof, or a pharmaceutically acceptable salt thereof has a unit dosage of 15 mg to 150 mg.

In some embodiments, an exemplary unit dosage of the compound of formula (I), a stereoisomer thereof, or a pharmaceutically acceptable salt thereof of the present disclosure includes, but is not limited to, 15 mg, 30 mg, 45 mg, 60 mg, 75 mg, 120 mg and 150 mg.

In some embodiments, the compound of formula (I), a stereoisomer thereof, or a pharmaceutically acceptable salt thereof is administered to the subject in a tablet or capsule form.

In some embodiments, the compound of formula (I), a stereoisomer thereof, or a pharmaceutically acceptable salt thereof is orally administered to the subject.

In some embodiments, an exemplary subject in the present disclosure includes, but is not limited to, a mammal.

In some embodiments, an exemplary mammal in the present disclosure includes, but is not limited to, a dog, cat, cow, sheep, horse and human.

In some embodiments, the subject is a human.

In some embodiments, the subject suffers from latent tuberculosis infection.

In some embodiments, the subject has a medical history of pulmonary tuberculosis infection, tuberculosis, and/or tuberculous pleurisy.

In some embodiments, the Sjögren's syndrome of the present disclosure is primary Sjögren's syndrome.

In some embodiments, the Sjögren's syndrome of the present disclosure is secondary Sjögren's syndrome.

In some embodiments, the compound of formula (I), a stereoisomer thereof, or a pharmaceutically acceptable salt thereof of the present disclosure can be used in the prevention or treatment of Sjögren's syndrome in a subject suffering from latent tuberculosis infection, and latent tuberculosis infection is not reactivated after treatment.

In some embodiments, the compound of formula (I), a stereoisomer thereof, or a pharmaceutically acceptable salt thereof of the present disclosure can be used in the prevention or treatment of Sjögren's syndrome in a subject having a medical history of pulmonary tuberculosis infection, tuberculosis, and/or tuberculous pleurisy, and pulmonary tuberculosis infection, tuberculosis, and/or tuberculous pleurisy is/are not reactivated after treatment.

In some embodiments, the compound of formula (I), a stereoisomer thereof, or a pharmaceutically acceptable salt thereof of the present disclosure does not require dose titration when used in the prevention or treatment of Sjögren's syndrome.

In some embodiments, the compound of formula (I), a stereoisomer thereof, or a pharmaceutically acceptable salt thereof of the present disclosure alleviates xerostomia in the subject when used in the prevention or treatment of Sjögren's syndrome.

In some embodiments, the compound of formula (I), a stereoisomer thereof, or a pharmaceutically acceptable salt thereof of the present disclosure alleviates xerophthalmia in the subject when used in the prevention or treatment of

Sjögren's syndrome.

In some embodiments, the compound of formula (I), a stereoisomer thereof, or a pharmaceutically acceptable salt thereof of the present disclosure improves the histopathology of minor salivary glands in the subject when used in the prevention or treatment of Sjögren's syndrome.

In some embodiments, the compound of formula (I), a stereoisomer thereof, or a pharmaceutically acceptable salt thereof of the present disclosure does not induce or exacerbate anxiety in the subject when used in the prevention or treatment of Sjögren's syndrome.

In some embodiments, the compound of formula (I), a stereoisomer thereof, or a pharmaceutically acceptable salt thereof of the present disclosure does not induce or exacerbate depression in the subject when used in the prevention or treatment of Sjögren's syndrome.

In yet another aspect, the present disclosure relates to a pharmaceutical composition for use in the prevention or treatment of Sjögren's syndrome in a subject, comprising a compound of formula (I), a stereoisomer thereof or a pharmaceutically acceptable salt thereof, and a pharmaceutically acceptable carrier, diluent or excipient,

Formula (I).

In some embodiments, the compound of formula (I), a stereoisomer thereof, or a pharmaceutically acceptable salt thereof is administered to the subject daily at a dose of 30 mg to 180 mg.

In some embodiments, the compound of formula (I), a stereoisomer thereof, or a pharmaceutically acceptable salt thereof is administered to the subject daily at a dose of 30 mg, 40 mg, 45 mg, 50 mg, 60 mg, 70 mg, 80 mg, 90 mg, 100 mg, 120 mg, 150 mg or 180 mg.

In some embodiments, the compound of formula (I), a stereoisomer thereof, or a pharmaceutically acceptable salt thereof is administered to the subject daily at a dose of 60 mg to 150 mg.

In some embodiments, the compound of formula (I), a stereoisomer thereof or a pharmaceutically acceptable salt thereof is administered to the subject daily at a dose of 30 mg, 45 mg, 60 mg, 120 mg or 150 mg.

In some embodiments, the compound of formula (I), a stereoisomer thereof, or a pharmaceutically acceptable salt thereof is administered to the subject daily at a dose of 150 mg.

In some embodiments, the compound of formula (I), a stereoisomer thereof, or a pharmaceutically acceptable salt thereof is administered to the subject daily at a dose of 120 mg.

In some embodiments, the compound of formula (I), a stereoisomer thereof, or a pharmaceutically acceptable salt thereof is administered to the subject at least once daily.

In some embodiments, the compound of formula (I), a stereoisomer thereof, or a pharmaceutically acceptable salt thereof is administered to the subject at least twice daily.

In some embodiments, the compound of formula (I), a stereoisomer thereof, or a pharmaceutically acceptable salt thereof is administered to the subject twice daily.

In some embodiments, the compound of formula (I), a stereoisomer thereof, or a pharmaceutically acceptable salt thereof is administered to the subject three times daily.

In some embodiments, the compound of formula (I), a stereoisomer thereof, or a pharmaceutically acceptable salt thereof is administered to the subject twice daily at a dose of 30 mg to 75 mg each time.

In some embodiments, the compound of formula (I), a stereoisomer thereof, or a pharmaceutically acceptable salt thereof is administered to the subject twice daily at a dose of 30 mg to 60 mg each time.

In some embodiments, the compound of formula (I), a stereoisomer thereof, or a pharmaceutically acceptable salt thereof has a unit dosage of 2.5 mg to 150 mg.

In some embodiments, an exemplary unit dosage of the compound of formula (I), a stereoisomer thereof, or a pharmaceutically acceptable salt thereof of the present disclosure includes, but is not limited to, 2.5 mg, 5 mg, 7.5 mg, 10 mg, 20 mg, 30 mg, 40 mg, 45 mg, 50 mg, 60 mg, 70 mg, 75 mg, 80 mg, 90 mg, 100 mg, 110 mg, 120 mg, 130 mg, 140 mg and 150 mg.

In some embodiments, the compound of formula (I), a stereoisomer thereof, or a pharmaceutically acceptable salt thereof has a unit dosage of 15 mg to 150 mg.

In some embodiments, an exemplary unit dosage of the compound of formula (I), a stereoisomer thereof, or a pharmaceutically acceptable salt thereof of the present disclosure includes, but is not limited to, 15 mg, 30 mg, 45 mg, 60 mg, 75 mg, 120 mg and 150 mg.

In some embodiments, the pharmaceutical composition comprising the compound of formula (I), a stereoisomer thereof, or a pharmaceutically acceptable salt thereof is in a tablet or capsule form.

In some embodiments, the pharmaceutical composition comprising the compound of formula (I), a stereoisomer thereof, or a pharmaceutically acceptable salt thereof is orally administered to the subject.

In some embodiments, an exemplary subject in the present disclosure includes, but is not limited to, a mammal.

In some embodiments, an exemplary mammal in the present disclosure includes, but is not limited to, a dog, cat, cow, sheep, horse and human.

In some embodiments, the subject is a human.

In some embodiments, the subject suffers from latent tuberculosis infection.

In some embodiments, the subject has a medical history of pulmonary tuberculosis infection, tuberculosis, and/or tuberculous pleurisy.

In some embodiments, the Sjögren's syndrome of the present disclosure is primary Sjögren's syndrome.

In some embodiments, the Sjögren's syndrome of the present disclosure is secondary Sjögren's syndrome.

In some embodiments, the compound of formula (I), a stereoisomer thereof, or a pharmaceutically acceptable salt thereof of the present disclosure can be used in the prevention or treatment of Sjögren's syndrome in a subject suffering from latent tuberculosis infection, and latent tuberculosis infection is not reactivated after treatment.

In some embodiments, the compound of formula (I), a stereoisomer thereof, or a pharmaceutically acceptable salt thereof of the present disclosure can be used in the prevention or treatment of Sjögren's syndrome in a subject having a medical history of pulmonary tuberculosis infection, tuberculosis, and/or tuberculous pleurisy, and pulmonary tuberculosis infection, tuberculosis, and/or tuberculous pleurisy is/are not reactivated after treatment.

In some embodiments, the compound of formula (I), a stereoisomer thereof, or a pharmaceutically acceptable salt thereof of the present disclosure does not require dose titration when used in the prevention or treatment of Sjögren's syndrome.

In some embodiments, the compound of formula (I), a stereoisomer thereof, or a pharmaceutically acceptable salt thereof of the present disclosure alleviates xerostomia in the subject when used in the prevention or treatment of Sjögren's syndrome.

In some embodiments, the compound of formula (I), a stereoisomer thereof, or a pharmaceutically acceptable salt thereof of the present disclosure alleviates xerophthalmia in the subject when used in the prevention or treatment of Sjögren's syndrome.

In some embodiments, the compound of formula (I), a stereoisomer thereof, or a pharmaceutically acceptable salt thereof of the present disclosure improves the histopathology of minor salivary glands in the subject when used in the prevention or treatment of Sjögren's syndrome.

In some embodiments, the compound of formula (I), a stereoisomer thereof, or a pharmaceutically acceptable salt thereof of the present disclosure does not induce or exacerbate anxiety in the subject when used in the prevention or treatment of Sjögren's syndrome.

In some embodiments, the compound of formula (I), a stereoisomer thereof, or a pharmaceutically acceptable salt thereof of the present disclosure does not induce or exacerbate depression in the subject when used in the prevention or treatment of Sjögren's syndrome.

The present disclosure will be illustrated in detail through the following examples in order to better understand various aspects and advantages of the present disclosure. However, it should be understood that the following examples are non-limiting and merely used to illustrate certain embodiments of the present disclosure.

**Example 1**

Preparation of compound of formula (I)

The compound of formula (I) was prepared with reference to the preparation method 2 of Example 3 in CN101885731A.

Example 2

Preparation of compound of formula (I) in S-configuration

The compound of formula (I) in S-configuration was prepared with reference to the preparation method of Example 1 in CN116332954A.

Example 3

Test in induced murine model of Sjögren's syndrome

1. Induced Modeling

Female C57BL/6 mice aged 6 to 8 weeks were sacrificed after cardiac perfusion for blood removal. The submandibular glands were isolated, and the envelope surrounding the glands was excised. The tissues were rinsed with normal saline containing 0.5 g/L sodium azide ($NaN_3$), minced, and homogenized with an equal volume of PBS buffer. The homogenate was subjected to five repeated freeze-thaw cycles at -80°C and ultrasonicated in ice-bath for 5 minutes to disrupt cells. The homogenate was then centrifuged in a high-speed refrigerated centrifuge (13,000 rpm, 10 minutes). The supernatant was collected to determine the content of protein antigens, i.e., the submandibular gland antigens. The antigen supernatant was diluted with PBS to 4 mg/mL and emulsified with an equal volume of Freund's complete adjuvant (oil phase injected into aqueous phase) to prepare a 2 mg/mL submandibular gland antigen emulsion.

The modeling animals were anesthetized, and subcutaneously injected with the prepared antigen emulsion at multiple points on the back of the modeling animals at a dose of 0.1 ml per mouse. The first immunization was designated as d0 (Day 0). Booster immunizations were performed on Days 7, 14, 21, 28, and 35 of the study, respectively, using the same induction method as the first immunization (the number of immunizations can be adjusted based on monitoring of saliva flow rate).

2. Grouping and Administration

Baseline determination of saliva flow rate of the experimental animals was performed prior to the first immunization (the detection method was described in detail hereinafter). The experimental animals were randomly divided into groups according to the saliva flow rate, and were administered on Day 1 after the first immunization, including a blank control group, a model control group, the Example 2 (60 mg/kg) group, the Example 2 (100 mg/kg) group, and the cyclophosphamide (30 mg/kg) group, with eight experimental animals in each group. The animals in the Example 2 groups were administered by intragastric gavage once daily, and the animals in the cyclophosphamide group were administered via intraperitoneal injection once every 2 days. The animals in each group were administered continuously for 6 weeks.

3. Monitoring indicators

3.1 Saliva Flow Rate

Mice were anesthetized via isoflurane inhalation, and a pre-weighed dry cotton ball was placed under the tongue of each mouse. Pilocarpine (5 mg/kg) was administered by intraperitoneal injection, followed by saliva collection for a period of 7 minutes. The cotton ball was then removed and weighed to obtain the wet weight. Saliva samples were collected from mice at Week 0 before the first immunization, as well as at Week 2, Week 4 and Week 6 after the first immunization. The saliva flow rate (mg/g/min) was calculated, and the change in saliva flow rate was observed.

Saliva flow rate (mg/g/min) = (wet weight of cotton ball - dry weight of cotton ball) / body weight / 7

3.3 Pathological Examination

One unilateral submandibular gland was harvested for pathological examination, while the remaining salivary glands and serum were used for detection of cytokines including IL-17, IL-6, and TNF-$\alpha$.

Pathological scoring criteria:

Microscopic observation was performed to evaluate the integrity of submandibular gland tissue structure, presence or absence of lymphocyte infiltration, and obvious abnormalities (such as dilation, edema, or lymphocytic foci formation) of blood vessels and glandular ducts. Scoring was carried out according to the degree of damage.

Score 0: No lymphocyte infiltration or scattered lymphocyte infiltration.

Score 1: Mild lymphocyte infiltration, no lymphocytic foci formation, and no other obvious abnormalities.

Score 2: Moderate lymphocyte infiltration, no lymphocytic foci formation, and mild edema of blood vessels and glandular ducts.

Score 3: One lymphocytic focus (infiltrating lymphocytes > 50) per 4 $mm^2$ field of view, with moderate edema and dilation of blood vessels and glandular ducts.

Score 4: 2 to 3 lymphocytic foci per 4 $mm^2$ field of view, with damage including acinar atrophy, and severe edema of blood vessels and glandular ducts.

4. The experimental protocol was detailed in the table below:

Table 1. Experimental protocol

<table>
<tr><th>Group</th><th>Numbers of mice</th><th>Administration dosage (mg/kg)</th><th>Administration route</th><th>Solvent</th><th>Administration period</th><th>Animal induction and treatment</th></tr>
<tr><td>Blank control</td><td>8</td><td>-</td><td>-</td><td>-</td><td>-</td><td rowspan="5">Induction:<br>Immunization was induced via subcutaneous (s.c.) injection on d0, d7, d14, d21, d28 and d35.<br>Antigen emulsion:<br>FCA + antigen (antigen concentration: 2 mg/ml after emulsification).<br>Collection and testing:<br>1) Saliva was collected at 0w, 4w, 6w and 8w to calculate saliva flow rate (mg/g/min).<br>2) Changes in water and food intake of the animals were monitored weekly.<br>3) A unilateral submandibular gland was harvested for pathological examination.</td></tr>
<tr><td>Model control</td><td>8</td><td>-</td><td rowspan="3">i.g.</td><td rowspan="3">1.0% CMC-Na solution</td><td rowspan="3">qd×42</td></tr>
<tr><td rowspan="2">Example 2</td><td>8</td><td>60</td></tr>
<tr><td>8</td><td>100</td></tr>
<tr><td>cyclophosphamide</td><td>8</td><td>30</td><td>i.p.</td><td>0.9% Sodium chloride injection</td><td>q2d×21</td></tr>
</table>

The immunization frequency and observation duration were adjusted based on the saliva flow rate indices of the animals, and the administration period was adjusted based on tolerance of the animals.

5. Data processing

Animal body weight-related data were statistically analyzed using the general linear model test in SPSS 22.0 software. Firstly, sphericity test was performed using the repeated-measures analysis of variance. If $P > 0.05$, the repeated measures data were considered uncorrelated, and one-way ANOVA was adopted for inter-group statistical analysis. If $P \leq 0.05$, the repeated measures data were considered correlated, and multivariate analysis of variance was applied for inter-group statistical analysis.

For saliva flow rate, Levene's Test in SPSS 22.0 software was performed to verify homogeneity of variance. If the variance was homogeneous ($P > 0.05$), one-way ANOVA was used for inter-group statistical analysis. If the variance was heterogeneous ($P \leq 0.05$), the Kruskal-Wallis nonparametric test was performed. If the result of the Kruskal-Wallis nonparametric test was significant ($P \leq 0.05$), pairwise intergroup comparisons were performed using the non-parametric Mann-Whitney U test.

6. Test Results

6.1 Changes in animal body weight

At the end of the test, the actual administration period for each dose group of the compound of Example 2 was qd×54, and the actual administration period for the cyclophosphamide group was q2d×27. The body weight of animals in each dosing group exhibited an increasing trend throughout this test, indicating favorable animal tolerance to the dosing regimen adopted in this test.

6.2 Measurements of saliva flow rate

The measurements of saliva flow rate showed that the model group had a significant difference in saliva flow rate compared with the blank control group on Day 46, indicating successful establishment of the animal model. The measurements of saliva flow rate were shown in Table 2.

Table 2. Changes in saliva flow rate of experimental animals in each group (mg/g/min, $\overline{X}\pm SD$)

| Group | Day -2 | Day 46 | Day 55 |
|---|---|---|---|
| Blank control | 0.570±0.102 | 0.617±0.145 | 0.600±0.167 |
| Model control | 0.569±0.113 | 0.487±0.115* | 0.429±0.121* |
| Example 2 (60 mg/kg) | 0.571±0.120 | 0.669±0.142## | 0.632±0.132## |
| Example 2 (100 mg/kg) | 0.570±0.139 | 0.515±0.094 | 0.565±0.082# |
| Cyclophosphamide (30 mg/kg) | 0.570±0.127 | 0.568±0.101 | 0.500±0.072 |
| Note: *p < 0.05 versus the blank control group; #p < 0.05 and ##p < 0.01 versus the model control group. | | | |

6.3 Pathological Examination

Table 3. Cytokine levels in submandibular gland tissue homogenate (pg/ml, $\overline{X}\pm SD$)

| Group | Submandibular gland | | |
|---|---|---|---|
| | IL-6 | TNF-α | IL-17A |
| Model control | 169.2±39.6 | 153.5±20.9 | 532.7±211.1 |
| Example 2 (60 mg/kg) | 75.7±40.6**### | 73.5±27.5## | 417.5±120.6 |
| Example 2 (100 mg/kg) | 68.1±24.7***### | 114.4±78.8 | 450.1±104.8 |
| Cyclophosphamide (30 mg/kg) | 126.7±46.4 | 138.1±27.6 | 643.9±169.0 |
| Note: *p<0.05, **p<0.01, ***p<0.001 versus the blank control group; #p<0.05, ##p<0.01, ###p<0.001 versus the model control group. | | | |

Histopathological examination was performed on submandibular gland tissue samples. The main pathological changes in the submandibular gland tissues of animals of this model were inflammatory cell infiltration and morphological deformation of glandular ducts, as reported in the related references. The pathological examination results of this test showed that the submandibular gland tissues in the blank control group exhibited intact tissue morphology, with no or mild local lymphocyte infiltration and no other abnormalities. In the model control group, morphological deformation and edema of glandular ducts and blood vessels were observed, with more lymphocyte and inflammatory cell infiltration, and lymphocytic foci formation was observed in the field of view, indicating obvious lesions. Further histopathological scoring results showed that the histopathological scores for the blank, model, Example 2 (60 mg/kg), Example 2 (100 mg/kg) and cyclophosphamide groups were 0.13, 2.50, 0.88, 1.25 and 1.00, respectively.

Example 4

Clinical Study in Patients

A multicenter, randomized, double-blind, placebo-controlled, parallel-group phase III clinical trial evaluating the

efficacy and safety in 305 subjects with moderate to severe chronic plaque psoriasis was conducted, among which 105 subjects were T-SPOT positive without active tuberculosis. Clinical observation and evaluation were conducted throughout a 16-week core treatment period, a 36-week extended treatment period, and a follow-up period (4 weeks). Anxiety and depression scales were used as the evaluation tools to observe and evaluate the anxiety and depression status of the subjects during the screening period, upon completion of the 16-week core period, and 4 weeks after the last administration.

The experimental results showed that, up to the completion of the clinical study, none of the 105 T-SPOT-positive subjects without active tuberculosis, 7 subjects with confirmed prior pulmonary tuberculosis, 76 subjects with a history of tuberculosis, and 1 subject with prior tuberculous pleurisy experienced tuberculosis reactivation. No tuberculosis-related clinical abnormalities were observed in chest X-ray or CT examination for all subjects.

Table 4. Depression scale scores in a Phase III clinical trial of moderate-to-severe plaque psoriasis

| | | Week 16 | | Week 56 | |
|---|---|---|---|---|---|
| | | Treatment group | Placebo group | Treatment group | Placebo group |
| Indicator | | (N=211) | (N=94) | (N=211) | (N=94) |
| HADS anxiety scores (points) | mean | 2.3 | 3.0 | 3.1 | 2.4 |
| HADS depression scores (points) | mean | 3.0 | 3.7 | 3.8 | 3.3 |

Up to the completion of the clinical study, there were no statistically significant differences ($P>0.05$) in the mean HADS anxiety scores and the mean HADS depression scores (± standard deviation) between the treatment group and placebo group. No adverse events of depression or anxiety were reported by the subjects, and no occurrence of depression or suicidal tendency associated with the study drug was observed.

Example 5

Clinical Study in Patients

A Phase I clinical trial enrolling a total of 36 healthy Chinese subjects was conducted, with multiple oral administrations of the compound of Example 2 at doses of 15 mg BID, 30 mg BID, 60 mg BID and 75 mg BID, and placebo, respectively. The clinical trial showed favorable safety and good tolerability.

In the present disclosure, relational terms such as "first" and "second" are used merely to distinguish one entity or operation from another entity or operation, and do not necessarily require or imply any such actual relationship or order between these entities or operations.

It can be understood from the foregoing that although specific embodiments of the present disclosure have been described for illustrative purposes, various modifications or variations can be made by a person skilled in the art without departing from the spirit and scope of the present disclosure. All such modifications or variations should fall within the scope of the claims appended to the present disclosure.

## Claims

**1.** A method for preventing or treating Sjögren's syndrome, comprising administering to a subject in need thereof a prophylactically or therapeutically effective amount of a compound of formula (I), a stereoisomer thereof, or a pharmaceutically acceptable salt thereof,

formula (I).

2. The method of claim 1, wherein the compound of formula (I), a stereoisomer thereof, or a pharmaceutically acceptable salt thereof is administered to the subject daily at a dose of 30 mg to 180 mg.

3. The method of claim 1 or 2, wherein the compound of formula (I), a stereoisomer thereof, or a pharmaceutically acceptable salt thereof is administered to the subject daily at a dose of 60 mg to 150 mg.

4. The method of any one of claims 1 to 3, wherein the compound of formula (I), a stereoisomer thereof, or a pharmaceutically acceptable salt thereof is administered to the subject daily at a dose of 150 mg.

5. The method of any one of claims 1 to 4, wherein the compound of formula (I), a stereoisomer thereof, or a pharmaceutically acceptable salt thereof is administered to the subject daily at a dose of 120 mg.

6. The method of any one of claims 1 to 5, wherein the compound of formula (I), a stereoisomer thereof, or a pharmaceutically acceptable salt thereof is administered to the subject at least once daily.

7. The method of any one of claims 1 to 5, wherein the compound of formula (I), a stereoisomer thereof, or a pharmaceutically acceptable salt thereof is administered to the subject at least twice daily.

8. The method of any one of claims 1 to 7, wherein the compound of formula (I), a stereoisomer thereof, or a pharmaceutically acceptable salt thereof is administered to the subject twice daily at a dose of 30 mg to 75 mg each time.

9. The method of any one of claims 1 to 8, wherein the compound of formula (I), a stereoisomer thereof, or a pharmaceutically acceptable salt thereof is administered to the subject twice daily at a dose of 30 mg to 60 mg each time.

10. The method of any one of claims 1 to 9, wherein the compound of formula (I), a stereoisomer thereof, or a pharmaceutically acceptable salt thereof has a unit dosage of 2.5 mg to 150 mg.

11. The method of any one of claims 1 to 10, wherein the compound of formula (I), a stereoisomer thereof, or a pharmaceutically acceptable salt thereof has a unit dosage of 15 mg to 150 mg.

12. The method of any one of claims 1 to 11, wherein the compound of formula (I), a stereoisomer thereof, or a pharmaceutically acceptable salt thereof has a unit dosage of 15 mg, 30 mg, 45 mg, 60 mg, 75 mg, 120 mg or 150 mg.

13. The method of any one of claims 1 to 12, wherein the compound of formula (I), a stereoisomer thereof, or a pharmaceutically acceptable salt thereof is administered to the subject in a tablet or capsule form.

14. The method of any one of claims 1 to 13, wherein the compound of formula (I), a stereoisomer thereof, or a pharmaceutically acceptable salt thereof is orally administered to the subject.

15. The method of any one of claims 1 to 14, wherein the subject is a mammal, preferably a human.

**16.** The method of any one of claims 1 to 15, wherein the subject suffers from latent tuberculosis infection.

**17.** The method of any one of claims 1 to 16, wherein the subject has a medical history of pulmonary tuberculosis infection, tuberculosis, and/or tuberculous pleurisy.

**18.** The method of any one of claims 1 to 17, wherein the treatment of the subject does not require dose titration.

**19.** The method of any one of claims 1 to 17, wherein the subject suffers from latent tuberculosis infection, and latent tuberculosis infection is not reactivated after treatment.

**20.** The method of any one of claims 1 to 17, wherein the subject has a medical history of pulmonary tuberculosis infection, tuberculosis, and/or tuberculous pleurisy, and pulmonary tuberculosis infection, tuberculosis, and/or tuberculous pleurisy is/are not reactivated after treatment.

**21.** The method of any one of claims 1 to 17, wherein xerostomia in the subject is alleviated.

**22.** The method of any one of claims 1 to 17, wherein xerophthalmia in the subject is alleviated.

**23.** The method of any one of claims 1 to 17, wherein a histopathology of minor salivary glands in the subject is improved.

**24.** The method of any one of claims 1 to 17, wherein anxiety or depression in the subject is not induced or exacerbated.

**25.** A compound of formula (I), a stereoisomer thereof, or a pharmaceutically acceptable salt thereof for use in the prevention or treatment of Sjögren's syndrome in a subject,

O
S
O
O
N
S
O
NH
O
O
O
O

formula (I).

**26.** The compound of formula (I), a stereoisomer thereof, or a pharmaceutically acceptable salt thereof for use of claim 25, wherein the compound of formula (I), a stereoisomer thereof, or a pharmaceutically acceptable salt thereof is administered to the subject daily at a dose of 30 mg to 180 mg.

**27.** The compound of formula (I), a stereoisomer thereof, or a pharmaceutically acceptable salt thereof for use of claim 25 or 26, wherein the compound of formula (I), a stereoisomer thereof, or a pharmaceutically acceptable salt thereof is administered to the subject daily at a dose of 60 mg to 150 mg.

**28.** The compound of formula (I), a stereoisomer thereof, or a pharmaceutically acceptable salt thereof for use of any one of claims 25 to 27, wherein the compound of formula (I), a stereoisomer thereof, or a pharmaceutically acceptable salt thereof is administered to the subject daily at a dose of 150 mg.

**29.** The compound of formula (I), a stereoisomer thereof, or a pharmaceutically acceptable salt thereof for use of any one of claims 25 to 28, wherein the compound of formula (I), a stereoisomer thereof, or a pharmaceutically acceptable salt thereof is administered to the subject daily at a dose of 120 mg.

**30.** The compound of formula (I), a stereoisomer thereof, or a pharmaceutically acceptable salt thereof for use of any one of claims 25 to 29, wherein the compound of formula (I), a stereoisomer thereof, or a pharmaceutically acceptable salt thereof is administered to the subject at least once daily.

**31.** The compound of formula (I), a stereoisomer thereof, or a pharmaceutically acceptable salt thereof for use of any one of claims 25 to 30, wherein the compound of formula (I), a stereoisomer thereof, or a pharmaceutically acceptable salt thereof is administered to the subject at least twice daily.

**32.** The compound of formula (I), a stereoisomer thereof, or a pharmaceutically acceptable salt thereof for use of any one of claims 25 to 31, wherein the compound of formula (I), a stereoisomer thereof, or a pharmaceutically acceptable salt thereof is administered to the subject twice daily at a dose of 30 mg to 75 mg each time.

**33.** The compound of formula (I), a stereoisomer thereof, or a pharmaceutically acceptable salt thereof for use of any one of claims 25 to 32, wherein the compound of formula (I), a stereoisomer thereof, or a pharmaceutically acceptable salt thereof is administered to the subject twice daily at a dose of 30 mg to 60 mg each time.

**34.** The compound of formula (I), a stereoisomer thereof, or a pharmaceutically acceptable salt thereof for use of any one of claims 25 to 33, wherein the compound of formula (I), a stereoisomer thereof, or a pharmaceutically acceptable salt thereof has a unit dosage of 2.5 mg to 150 mg.

**35.** The compound of formula (I), a stereoisomer thereof, or a pharmaceutically acceptable salt thereof for use of any one of claims 25 to 34, wherein the compound of formula (I), a stereoisomer thereof, or a pharmaceutically acceptable salt thereof has a unit dosage of 15 mg to 150 mg.

**36.** The compound of formula (I), a stereoisomer thereof, or a pharmaceutically acceptable salt thereof for use of any one of claims 25 to 35, wherein the compound of formula (I), a stereoisomer thereof, or a pharmaceutically acceptable salt thereof has a unit dosage of 15 mg, 30 mg, 45 mg, 60 mg, 75 mg, 120 mg, or 150 mg.

**37.** The compound of formula (I), a stereoisomer thereof, or a pharmaceutically acceptable salt thereof for use of any one of claims 25 to 36, wherein the compound of formula (I), a stereoisomer thereof, or a pharmaceutically acceptable salt thereof is administered to the subject in a tablet or capsule form.

**38.** The compound of formula (I), a stereoisomer thereof, or a pharmaceutically acceptable salt thereof for use of any one of claims 25 to 37, wherein the compound of formula (I), a stereoisomer thereof, or a pharmaceutically acceptable salt thereof is orally administered to the subject.

**39.** The compound of formula (I), a stereoisomer thereof, or a pharmaceutically acceptable salt thereof for use of any one of claims 25 to 38, wherein the subject is a mammal, preferably a human.

**40.** The compound of formula (I), a stereoisomer thereof, or a pharmaceutically acceptable salt thereof for use of any one of claims 25 to 39, wherein the subject suffers from latent tuberculosis infection.

**41.** The compound of formula (I), a stereoisomer thereof, or a pharmaceutically acceptable salt thereof for use of any one of claims 25 to 40, wherein the subject has a medical history of pulmonary tuberculosis infection, tuberculosis, and/or tuberculous pleurisy.

**42.** The compound of formula (I), a stereoisomer thereof, or a pharmaceutically acceptable salt thereof for use of any one of claims 25 to 41, wherein the treatment of the subject does not require dose titration.

**43.** The compound of formula (I), a stereoisomer thereof, or a pharmaceutically acceptable salt thereof for use of any one of claims 25 to 41, wherein the subject suffers from latent tuberculosis infection, and latent tuberculosis infection is not reactivated after treatment.

**44.** The compound of formula (I), a stereoisomer thereof, or a pharmaceutically acceptable salt thereof for use of any one of claims 25 to 41, wherein the subject has a medical history of pulmonary tuberculosis infection, tuberculosis, and/or tuberculous pleurisy, and pulmonary tuberculosis infection, tuberculosis, and/or tuberculous pleurisy is/are not reactivated after treatment.

**45.** The compound of formula (I), a stereoisomer thereof, or a pharmaceutically acceptable salt thereof for use of any one of claims 25 to 41, wherein xerostomia in the subject is alleviated.

**46.** The compound of formula (I), a stereoisomer thereof, or a pharmaceutically acceptable salt thereof for use of any one

of claims 25 to 41, wherein xerophthalmia in the subject is alleviated.

**47.** The compound of formula (I), a stereoisomer thereof, or a pharmaceutically acceptable salt thereof for use of any one of claims 25 to 41, wherein a histopathology of minor salivary glands in the subject is improved.

**48.** The compound of formula (I), a stereoisomer thereof, or a pharmaceutically acceptable salt thereof for use of any one of claims 25 to 41, wherein anxiety or depression in the subject is not induced or exacerbated.

**49.** Use of a compound of formula (I), a stereoisomer thereof, or a pharmaceutically acceptable salt thereof in the preparation of a medicament for the prevention or treatment of Sjögren's syndrome in a subject,

formula (I).

**50.** The use of claim 49, wherein the compound of formula (I), a stereoisomer thereof, or a pharmaceutically acceptable salt thereof is administered to the subject daily at a dose of 30 mg to 180 mg.

**51.** The use of claim 49 or 50, wherein the compound of formula (I), a stereoisomer thereof, or a pharmaceutically acceptable salt thereof is administered to the subject daily at a dose of 60 mg to 150 mg.

**52.** The use of any one of claims 49 to 51, wherein the compound of formula (I), a stereoisomer thereof, or a pharmaceutically acceptable salt thereof is administered to the subject daily at a dose of 150 mg.

**53.** The use of any one of claims 49 to 52, wherein the compound of formula (I), a stereoisomer thereof, or a pharmaceutically acceptable salt thereof is administered to the subject daily at a dose of 120 mg.

**54.** The use of any one of claims 49 to 53, wherein the compound of formula (I), a stereoisomer thereof, or a pharmaceutically acceptable salt thereof is administered to the subject at least once daily.

**55.** The use of any one of claims 49 to 54, wherein the compound of formula (I), a stereoisomer thereof, or a pharmaceutically acceptable salt thereof is administered to the subject at least twice daily.

**56.** The use of any one of claims 49 to 55, wherein the compound of formula (I), a stereoisomer thereof, or a pharmaceutically acceptable salt thereof is administered to the subject twice daily at a dose of 30 mg to 75 mg each time.

**57.** The use of any one of claims 49 to 56, wherein the compound of formula (I), a stereoisomer thereof, or a pharmaceutically acceptable salt thereof is administered to the subject twice daily at a dose of 30 mg to 60 mg each time.

**58.** The use of any one of claims 49 to 57, wherein the compound of formula (I), a stereoisomer thereof, or a pharmaceutically acceptable salt thereof has a unit dosage of 2.5 mg to 150 mg.

**59.** The use of any one of claims 49 to 58, wherein the compound of formula (I), a stereoisomer thereof, or a pharmaceutically acceptable salt thereof has a unit dosage of 15 mg to 150 mg.

**60.** The use of any one of claims 49 to 59, wherein the compound of formula (I), a stereoisomer thereof, or a

pharmaceutically acceptable salt thereof has a unit dosage of 15 mg, 30 mg, 45 mg, 60 mg, 75 mg, 120 mg, or 150 mg.

**61.** The use of any one of claims 49 to 60, wherein the compound of formula (I), a stereoisomer thereof, or a pharmaceutically acceptable salt thereof is administered to the subject in a tablet or capsule form.

**62.** The use of any one of claims 49 to 61, wherein the compound of formula (I), a stereoisomer thereof, or a pharmaceutically acceptable salt thereof is orally administered to the subject.

**63.** The use of any one of claims 49 to 62, wherein the subject is a mammal, preferably a human.

**64.** The use of any one of claims 49 to 63, wherein the subject suffers from latent tuberculosis infection.

**65.** The use of any one of claims 49 to 64, wherein the subject has a medical history of pulmonary tuberculosis infection, tuberculosis, and/or tuberculous pleurisy.

**66.** The use of any one of claims 49 to 65, wherein the treatment of the subject does not require dose titration.

**67.** The use of any one of claims 49 to 65, wherein the subject suffers from latent tuberculosis infection, and latent tuberculosis infection is not reactivated after treatment.

**68.** The use of any one of claims 49 to 65, wherein the subject has a medical history of pulmonary tuberculosis infection, tuberculosis, and/or tuberculous pleurisy, and pulmonary tuberculosis infection, tuberculosis, and/or tuberculous pleurisy is/are not reactivated after treatment.

**69.** The use of any one of claims 49 to 65, wherein xerostomia in the subject is alleviated.

**70.** The use of any one of claims 49 to 65, wherein xerophthalmia in the subject is alleviated.

**71.** The use of any one of claims 49 to 65, wherein a histopathology of minor salivary glands in the subject is improved.

**72.** The use of any one of claims 49 to 65, wherein anxiety or depression in the subject is not induced or exacerbated.

| INTERNATIONAL SEARCH REPORT | International application No. **PCT/CN2024/132140** |
|---|---|

**A. CLASSIFICATION OF SUBJECT MATTER**

A61K31/407(2006.01)i； A61K9/48(2006.01)i； A61K9/20(2006.01)i； A61P37/02(2006.01)i

According to International Patent Classification (IPC) or to both national classification and IPC

**B. FIELDS SEARCHED**

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

CNTXT, WPABS, ENTXT, VEN, WOTXT, USTXT, EPTXT, CJFD, CNKI, ISI-WEB OF SCIENCE, STN: 噻吩, 吡咯, 砜, 磺酰, 苯, 干燥综合征, 口干, 眼干, 基于式(I)进行了结构检索; PDE, TNF, thiophene, pyrrol+, +sulfonyl, phenyl, search based on the structure of formula (I), sjogren, oral, eye

**C. DOCUMENTS CONSIDERED TO BE RELEVANT**

| Category* | Citation of document, with indication, where appropriate, of the relevant passages | Relevant to claim No. |
|---|---|---|
| X | CN 111821299 A (TIANJIN HEMAY PHARMACEUTICAL SCI-TECH CO., LTD.) 27 October 2020 (2020-10-27)<br>description, paragraphs [0052], [0055]-[0056], [0065] and [0085] | 25-48 |
| X | CN 108059635 A (TIANJIN HEMAY PHARMACEUTICAL SCI-TECH CO., LTD.) 22 May 2018 (2018-05-22)<br>claims 6 and 9-10 | 25-48 |
| A | CN 111821299 A (TIANJIN HEMAY PHARMACEUTICAL SCI-TECH CO., LTD.) 27 October 2020 (2020-10-27)<br>claims 1 and 5 | 49-72 |
| A | CN 108059635 A (TIANJIN HEMAY PHARMACEUTICAL SCI-TECH CO., LTD.) 22 May 2018 (2018-05-22)<br>claims 6 and 9-10 | 49-72 |
| A | CN 101186612 A (TIANJIN HEMAY BIO-TECH CO., LTD.) 28 May 2008 (2008-05-28)<br>claims 1-17 | 25-72 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **13 December 2024** | **31 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No. **PCT/CN2024/132140**

**C. DOCUMENTS CONSIDERED TO BE RELEVANT**

| Category* | Citation of document, with indication, where appropriate, of the relevant passages | Relevant to claim No. |
|---|---|---|
| A | CN 107007606 A (NANJING SHUPENG BIOTECHNOLOGY CO., LTD.) 04 August 2017 (2017-08-04)<br>claims 1-5 | 25-72 |
| A | CN 1420889 A (CELGENE CORP.) 28 May 2003 (2003-05-28)<br>embodiments 8, 24 and 26, and claims 1 and 34 | 25-72 |

Form PCT/ISA/210 (second sheet) (July 2022)

**INTERNATIONAL SEARCH REPORT**

International application No.
**PCT/CN2024/132140**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **1-24**
because they relate to subject matter not required to be searched by this Authority, namely:

The subject matter of claims 1-24 is a method for treating diseases, which belongs to subject matter for which no search is required by the International Searching Authority as defined in PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/132140**

| Patent document cited in search report | Publication date (day/month/year) | Patent family member(s) | Publication date (day/month/year) |
|---|---|---|---|
| CN 111821299 A | 27 October 2020 | None | |
| CN 108059635 A | 22 May 2018 | None | |
| CN 101186612 A | 28 May 2008 | EP 2083011 A1 | 29 July 2009 |
| | | EP 2083011 A4 | 09 March 2011 |
| | | EP 2083011 B1 | 04 June 2014 |
| | | ES 2498797 T3 | 25 September 2014 |
| | | BRPI 0716005 A2 | 13 May 2014 |
| | | JP 2010509377 A | 25 March 2010 |
| | | JP 5491863 B2 | 14 May 2014 |
| | | WO 2008058448 A1 | 22 May 2008 |
| | | US 2010179189 A1 | 15 July 2010 |
| | | US 8466191 B2 | 18 June 2013 |
| | | KR 20090086592 A | 13 August 2009 |
| | | AU 2007321636 A1 | 22 May 2008 |
| | | MX 2009005222 A | 02 July 2009 |
| | | CA 2669357 A1 | 22 May 2008 |
| | | RU 2009121676 A | 20 December 2010 |
| | | RU 2473551 C2 | 27 January 2013 |
| CN 107007606 A | 04 August 2017 | WO 2017133523 A1 | 10 August 2017 |
| | | EP 3412292 A1 | 12 December 2018 |
| | | EP 3412292 A4 | 27 February 2019 |
| | | EP 3412292 B1 | 18 September 2019 |
| | | EP 3412292 B8 | 13 November 2019 |
| | | US 2019030024 A1 | 31 January 2019 |
| | | US 10702519 B2 | 07 July 2020 |
| | | JP 2019503401 A | 07 February 2019 |
| | | JP 6638092 B2 | 29 January 2020 |
| CN 1420889 A | 28 May 2003 | ATE 544452 T1 | 15 February 2012 |
| | | MXPA 02004793 A | 14 October 2003 |
| | | ES 2380574 T3 | 16 May 2012 |
| | | NZ 519459 A | 28 November 2003 |
| | | EP 2263669 A1 | 22 December 2010 |
| | | EP 2263669 B1 | 08 February 2012 |
| | | PT 2263669 E | 23 April 2012 |
| | | CY 1112707 T1 | 10 February 2016 |
| | | FI 20020892 A0 | 10 May 2002 |
| | | FI 20020892 A | 20 June 2002 |
| | | FI 119931 B | 15 May 2009 |
| | | HK 1049158 A1 | 02 May 2003 |
| | | ATE 506058 T1 | 15 May 2011 |
| | | CA 2392081 A1 | 17 May 2001 |
| | | CA 2392081 C | 05 January 2010 |
| | | DE 60034139 D1 | 10 May 2007 |
| | | DE 60034139 T2 | 06 December 2007 |
| | | HK 1053467 A1 | 24 October 2003 |
| | | US 6667316 B1 | 23 December 2003 |
| | | NO 20022223 D0 | 08 May 2002 |
| | | NO 20022223 L | 08 July 2002 |
| | | NO 323633 B1 | 18 June 2007 |
| | | AU 1478001 A | 06 June 2001 |

Form PCT/ISA/210 (patent family annex) (July 2022)

**INTERNATIONAL SEARCH REPORT**
**Information on patent family members**

International application No.
**PCT/CN2024/132140**

| Patent document cited in search report | Publication date (day/month/year) | Patent family member(s) | | | Publication date (day/month/year) |
|---|---|---|---|---|---|
| | | AU | 782409 | B2 | 28 July 2005 |
| | | ES | 2381174 | T3 | 23 May 2012 |
| | | EP | 2255801 | A1 | 01 December 2010 |
| | | EP | 2255801 | B1 | 08 February 2012 |
| | | JP | 2004500346 | A | 08 January 2004 |
| | | JP | 5116201 | B2 | 09 January 2013 |
| | | WO | 0134606 | A1 | 17 May 2001 |
| | | ES | 2282147 | T3 | 16 October 2007 |
| | | EP | 1228071 | A1 | 07 August 2002 |
| | | EP | 1228071 | A4 | 04 December 2002 |
| | | EP | 1228071 | B1 | 28 March 2007 |
| | | EP | 1228071 | B8 | 29 February 2012 |
| | | ATE | 357913 | T1 | 15 April 2007 |
| | | SI | 1228071 | T1 | 31 August 2007 |
| | | CY | 1107610 | T1 | 13 March 2013 |
| | | EP | 1698334 | A1 | 06 September 2006 |
| | | EP | 1698334 | B1 | 20 April 2011 |
| | | ATE | 544451 | T1 | 15 February 2012 |
| | | PT | 1228071 | E | 31 May 2007 |
| | | ES | 2363933 | T3 | 19 August 2011 |
| | | DK | 2263669 | T3 | 23 April 2012 |
| | | DK | 1228071 | T3 | 06 August 2007 |
| | | AU | 2005202964 | A1 | 11 August 2005 |
| | | AU | 2005202964 | B2 | 22 November 2007 |
| | | NO | 20071490 | L | 08 July 2002 |
| | | NO | 336210 | B1 | 15 June 2015 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311516190
- CN 101885731 A
- CN 116332954 A